# EUROPEAN PATENT APPLICATION

(11) **EP 4 382 096 A1**
(43) Date of publication of application: **12.06.2024**
(21) Application number: 21952304.0
(22) Date of filing: 05.08.2021
(51) Int. Cl.: A61K 9/19, A61K 31/495, A61P 25/24

(54) **LYOPHILIZED VORTIOXETINE PAMOATE POWDER FOR INJECTION AND PREPARATION METHOD THEREFOR**

(71) Applicant: Zhejiang Huahai Pharmaceutical Co., Ltd., Taizhou, Zhejiang 317024 (CN)
(72) Inventor: LIU, Tengfei, Zhejiang 317024 (CN); FANG, Jialei, Zhejiang 317024 (CN); WANG, Yanan, Zhejiang 317024 (CN); NI, Laiyi, Zhejiang 317024 (CN)
(74) Representative: dompatent von Kreisler Selting Werner - Partnerschaft von Patent- und Rechtsanwälten mbB
(86) International application number: PCT/CN2021/110851
(87) International publication number: WO 2023/010410

(57) **Abstract**

Provided are a lyophilized vortioxetine pamoate powder for injection and a preparation method therefor. The prepared powder for injection product has good permeability and stability, and is very suitable for industrialization.

## Description

### TECHNICAL FIELD

The present application pertains to the field of pharmaceutical research and development, in particular to a method for preparing a suspension of vortioxetine pamoate, and to a method for preparing a lyophilized vortioxetine pamoate powder for injection.

### BACKGROUND

Vortioxetine (chemically named as 1-[2-(2,4-dimethylphenylsulfanyl)phenyl]piperazine) is an antidepressant jointly developed by Takeda Pharmaceuticals and Lundbeck Pharmaceuticals, the structural formula of which is as follows:

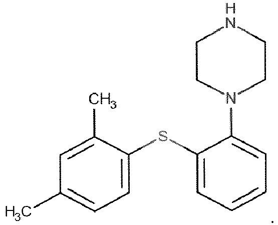

Vortioxetine can effectively overcome some shortcomings of common antidepressants, such as weight gain, drowsiness, medicament sedation, and other adverse reactions, and has the advantages of multiple targets, low adverse reaction rate, low relapse rate, safety and tolerance for continuous treatment, and the like. Currently, the marketed ones are vortioxetine hydrobromide tablets, which have a shorter duration of efficacy in the body and need to be taken once a day.

It is disclosed in WO2017167180A1 that, for the first time, vortioxetine and pamoic acid were made into a salt in a certain ratio, which prolonged the residence time of vortioxetine in vivo and thus achieved a slow release of vortioxetine in vivo. Vortioxetine pamoate was prepared into a stable long-acting preparation, allowing the administration to the patient once every two or four weeks, thereby greatly improving the utilization efficiency of the medicament, improving the therapeutic efficacy of the medicament, improving the patient's compliance and reducing adverse reactions.

It is mentioned in WO2017167180A1 that the vortioxetine pamoate was administered as a suspension, wherein the pharmaceutically acceptable carrier is preferably a viscous injectable carrier, for example, a viscous injectable carrier having a viscosity of at least 20 cp at 20 °C. High viscosity results in bigger difficulty in preparing the suspension before clinical use, higher force to withdraw the suspension into the syringe after preparation, and higher requirements for operators. In addition, if vortex is needed in preparation (such as a vortex for 2 min), it is required to use an external equipment such as a vortex machine, which increases the difficulty and time of preparation. For the preparation of a suspension, even if a uniformly mixed suspension can be obtained eventually, the operation process may be complex, time-consuming and laborious, and there is still a risk that the API may form into small aggregates that are difficult to be dispersed.

### SUMMARY OF THE INVENTION

An object of the present application is to provide a vortioxetine pamoate powder for injection and a preparation method therefor, which is realized by the following technical embodiments.

### Firstly, the present application provides a powder for injection consisting of vortioxetine pamoate and pharmaceutically acceptable excipients

In some embodiments of the present application, the mass percentage of the vortioxetine pamoate is 54.0% to 72.4%, based on total mass of the composition.

In some embodiments of the present application, the pharmaceutically acceptable excipients comprise a suspending agent selected from the group consisting of a low molecular weight suspending agent and a high molecular weight suspending agent, including glycerol, arabic gum, agar, tragacanth gum, methyl cellulose, sodium carboxymethyl cellulose, hydroxypropyl cellulose, povidone, glucan, carbopol, aluminum monostearate, and the like. The suspension agent accounts for 2.3% to 13.8% (w/w), preferably 6.9% to 10.8% (w/w) of the total mass of the powder for injection. The suspending agent is preferably sodium carboxymethyl cellulose, more preferably sodium carboxymethyl cellulose having a viscosity of 50 cp to 200 cp (4%, w/v).

In some embodiments of the present application, the pharmaceutically acceptable excipients further comprise a freeze-drying protective agent selected from the group consisting of saccharides and polyols, such as sucrose, trehalose, lactose, xylitol, glucose or mannitol. Preferably, the freeze-drying protective agent accounts for 4.6% to 35.0% (w/w) of the total mass of the powder for injection.

In some embodiments of the present application, the pharmaceutically acceptable excipients further comprise an osmotic pressure adjuster selected from the group consisting of sodium chloride, potassium chloride, mannitol, glucose, borax, and the like.

In some embodiments of the present application, the mannitol is preferred as the osmotic pressure adjuster and the freeze-drying protective agent, and the injection grade mannitol is further preferred. Mannitol accounts for 20.7% to 32.5% (w/w) of the total mass of the powder for injection.

In some embodiments of the present application, the pharmaceutically acceptable excipients are preferably free of a wetting agent, such as polysorbate (also known as Tween) and poloxamer. Wetting agent, playing a dispersing function in a formulation, can improve the mixing uniformity of a suspension. However, the inventors unexpectedly found that the prepared suspension will settle rapidly if a wetting agent such as polysorbate is added to the formulation of the present application.

The preferred formulation of the present application consists of vortioxetine pamoate monohydrate, sodium carboxymethyl cellulose, mannitol and water for injection.

The preferred preparation specification of the present application is 140 mg to 280 mg, which is equivalent to about 235.5 mg to 471.0 mg of vortioxetine pamoate monohydrate, wherein, preferably, the amount of sodium carboxymethyl cellulose is 6.9% to 10.8% (w/w), and the amount of mannitol is 20.7% to 32.5% (w/w).

The components and amounts of the preferred vortioxetine pamoate powder for injection of the present application are as follows:

| Component | Amount percentage (w/w) |
|---|---|
| Vortioxetine pamoate monohydrate | 56.7% to 72.4% |

| Sodium carboxymethyl cellulose | 6.9% to 10.8% |
|---|---|
| Mannitol | 20.7% to 32.5% |

In some embodiments of the present application, the vortioxetine pamoate has a particle size of 1 µm to 40 µm (D90), preferably 1 µm to 20 µ m (D90), more preferably 1 µm to 10 µm (D90).

D90 refers to the corresponding particle size when the cumulative particle size distribution of the sample measured by a laser granulometer reaches 90%.

The present application further provides a preparation method of vortioxetine pamoate powder for injection, comprising the following steps:
(1) preparation of a diluent solution: heating a water for injection, adding excipients under stirring, stirring until being completely dissolved and filtering;
(2) a primary mixing: weighing vortioxetine pamoate monohydrate powders, slowly adding into the diluent solution obtained in step (1) under stirring, and performing a high-shear dispersion to obtain a primary mixed solution;
(3) a secondary mixing: feeding the primary mixed solution obtained in step (2) into a homogenizer, performing a homogenization to obtain a suspension having a particle size of 1 µm to 40 µm (D90), filling and half-stoppering to obtain a filled sample; and
(4) freeze-drying: placing the filled sample obtained in step (3) into a dryer, freeze-drying under set freeze-drying parameters, and stoppering.

The particle size in step (3) is preferably 1 µm to 20 µm (D90), more preferably 1 µm to 10 µm (D90).

Particle size has a great influence on the properties, syringeability and bioavailability of the product. A wide particle size distribution or a poor mixing uniformity will lead to caking and sedimentation, resulting in needle blockage. In the preparation process of the present application, a high-shear mixing machine and a high-pressure homogenizer are used to adjust the particle size to the desired range.

This step provides a method of preparing a lyophilized formulation of vortioxetine pamoate, comprising the following steps:
(1) freezing the filled suspension of the vortioxetine pamoate to -40°C;
(2) performing a primary drying at a temperature lower than about 0°C under vacuum; and
(3) performing a secondary drying at a temperature higher than about 0°C.

The inventive concepts of the present application are as follows.
1. The flocculation effect of the product is reduced to ensure that no significant sedimentation occurs when the suspension is placed by optimizing the preparation and formulation of the product.
2. The high-shear mixing and high-pressure homogenization processes are determined to prepare a uniform suspension having a suitable particle size through exploration and study.
3. The pre-freezing, primary drying and secondary drying processes are optimized respectively to successfully prepare the powder for injection having qualified physical and chemical properties such as appearance and moisture based on the key freeze-drying parameters.

The powder for injection prepared according to the present application has uniformly dispersed particles and normal appearance, and the internal structure of solid matter in the broken vial is uniform without shrinkage. After adding water for injection, the powder for injection can be quickly reconstructed (a uniformly mixed suspension can be obtained after a gentle shake for less than30 s), and the reconstructed solution can pass through a needle with a size of 19 G to 22 G, indicating a good permeability. The development of this preparation not only improves the feasibility of industrialization, but also reduces the difficulties in medicament transportation and storage, thereby providing a more effective and convenient treatment for clinical mental patients.

### DESCRIPTION OF THE DRAWINGS

In order to explain the Examples of the present application and the technical solutions of the prior art more clearly, the following briefly introduces the drawings used in the Examples and the prior art. Obviously, the drawings in the following description are only some Examples of the present application, and for those skilled in the art, other drawings may be obtained based on these drawings.
FIG. 1: Investigation of the influence of samples stability.
FIG. 2: Investigation of the influence of particle size on samples stability.

The ordinate TSI is the stability index, which is an evaluation of the stability of the whole dispersion system, and the stability decreases with the increase of the value of TSI.

### DETAILED DESCRIPTION

The present application will be further described in detail below in conjunction with Examples, so that those skilled in the art can understand and practice the present application more clearly. However, the present application is not limited to the following Examples, and any changes, modifications and equivalent substitutions made to the present application without departing from the spirit and scope of the present application are within the scope of the present application.

### Example 1

| Component | Amount | Function |
|---|---|---|
| Vortioxetine pamoate monohydrate | 94.2 g | Active ingredient |
| Sodium carboxymethyl cellulose | 9 g | Suspending agent |
| Mannitol | 27 g | Osmotic pressure adjuster, freeze-drying protective agent |
| Polysorbate 80 | 1.2 g | Wetting agent |
| Water for injection | 1000 mL | Solvent |

| | | |
|---|---|---|
| Note: mannitol is of injection grade, and the sodium carboxymethyl cellulose having a viscosity of 50 cp to 200 cp (4%, w/v) is preferred. | | |

Process steps:
(1) 80% water for injection was taken and placed under a water bath at 50°C. Then mannitol and sodium carboxymethyl cellulose and polysorbate 80 were added in turn under stirring at 360 rpm until complete dissolution. The solution was cooled to room temperature, then weighed, and filtered with 0.22 µm microporous membrane to obtain a diluent solution.
(2) the active pharmaceutical ingredient was slowly added into the diluent solution by a high-shear mixing machine at 14,000 rpm. After the addition was completed, the resultant was mixed for 10 minutes to obtain an intermediate solution.
(3) the intermediate solution was put into a high-pressure homogenizer after the liquid flow rate was set. The pressure was slowly increased to 100 bar, at which the homogenization was performed for 3 cycles, and then slowly increased to 500 bar, at which the homogenization was performed for 2 cycles to obtain a suspension with a particle size of 10 µm (D90). The suspension was filled and half-stoppered.
(4) freeze-drying: the sample was put into a drying oven for freeze-drying, stoppered, taken out of the oven, and capped to obtain the vortioxetine powder for injection.

### Example 2

| Component | Amount | Function |
|---|---|---|
| Vortioxetine pamoate monohydrate | 94.2 g | Active ingredient |
| Sodium carboxymethyl cellulose | 9 g | Suspending agent |
| Mannitol | 27 g | Osmotic pressure adjuster, freeze-drying protective agent |
| Water for injection | 1000 mL | Solvent |

| | | |
|---|---|---|
| Note: mannitol is of injection grade, and the sodium carboxymethyl cellulose having a viscosity of 50 cp to 200 cp (4%, w/v) is preferred. | | |

Process steps:
(1) 80% water for injection was taken and placed under a water bath at 50°C. Then mannitol and sodium carboxymethyl cellulose were added in turn under stirring at 360 rpm until complete dissolution. The solution was cooled to room temperature, then weighed, and filtered with 0.22 µm microporous membrane to obtain a diluent solution.
(2) the active pharmaceutical ingredient was slowly added into the diluent solution by a high-shear mixing machine at 14,000 rpm. After the addition was completed, the resultant was mixed for 10 minutes to obtain an intermediate solution.
(3) the intermediate solution was put into a high-pressure homogenizer after the liquid flow rate was set. The pressure was slowly increased to 100 bar, at which the homogenization was performed for 3 cycles, and then slowly increased to 500 bar, at which the homogenization was performed for 2 cycles to obtain a suspension with a particle size of 10 µm (D90). The suspension was filled and half-stoppered.
(4) freeze-drying: the sample was put into a drying oven for freeze-drying, stoppered, taken out of the oven, and capped to obtain the vortioxetine powder for injection.

### Example 3

| Component | Amount | Function |
|---|---|---|
| Vortioxetine pamoate monohydrate | 42.3 g | Active ingredient |
| Sodium carboxymethyl cellulose | 9 g | Suspending agent |
| Mannitol | 27 g | Osmotic pressure adjuster, freeze-drying protective agent |
| Water for injection | 1000 mL | Solvent |

| | | |
|---|---|---|
| Note: mannitol is of injection grade, and the sodium carboxymethyl cellulose having a viscosity of 50 cp to 200 cp (4%, w/v) is preferred. | | |

Process steps:
(1) 80% water for injection was taken and placed under a water bath at 50°C. Then mannitol and sodium carboxymethyl cellulose were added in turn under stirring at 360 rpm until complete dissolution. The solution was cooled to room temperature, then weighed, and filtered with 0.22 µm microporous membrane to obtain a diluent solution.
(2) the active pharmaceutical ingredient was slowly added into the diluent solution by a high-shear mixing machine at 14,000 rpm. After the addition was completed, the resultant was mixed for 10 minutes to obtain the intermediate solution.
(3) the intermediate solution was put into a high-pressure homogenizer after the liquid flow rate was set. The pressure was slowly increased to 100 bar, at which the homogenization was performed for 3 cycles, and then slowly increased to 500 bar, at which the homogenization was performed for 2 cycles to obtain a suspension with a particle size of 10 µm (D90). The suspension was filled and half-stoppered.
(4) freeze-drying: the sample was put into a drying oven for freeze-drying, stoppered, taken out of the oven, and capped to obtain the vortioxetine powder for injection.

### Example 4: Investigation of the influence of sample stability

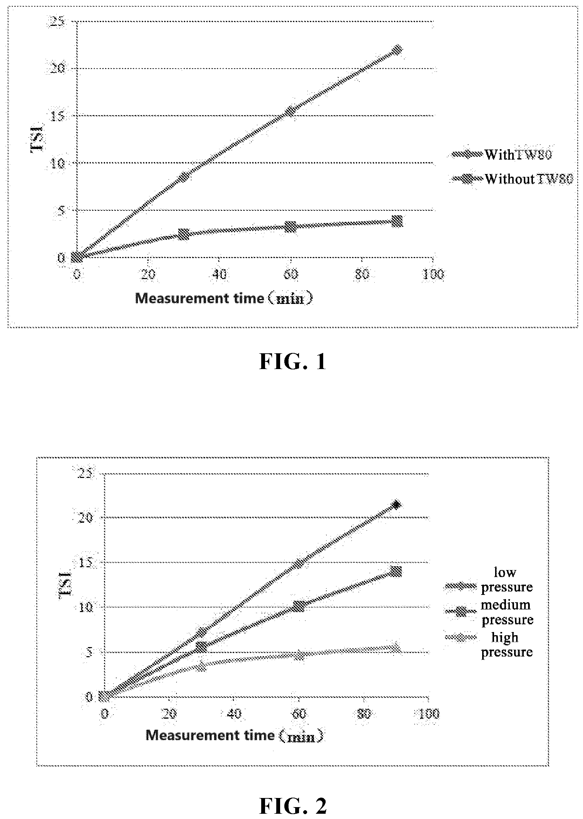

The product obtained in Example 1 (with polysorbate 80, referred to as TW80) and the product obtained in Example 2 (without TW80) were analyzed by a stability analyzer (model: TURBISCAN Lab) for the relationship between time and sedimentation, and the results are shown in FIG. 1.

The experimental results showed that for the system without polysorbate 80, the sedimentation rate of the active pharmaceutical ingredient particles was low. For the system introduced with polysorbate 80, the sedimentation rate of the active pharmaceutical ingredient particles was high and the layering phenomenon was obvious. The results showed that the properties of the suspensions of the two different systems were quite different, and the medicament solution without polysorbate 80 was better than that with polysorbate 80.

### Example 5: Investigation on the influence of particle size on sample stability

With reference to Example 2, samples with different particle sizes were obtained by changing the working pressure of the high-pressure homogenizer in step (3). The relationship between time and sedimentation was analyzed by stability analyzer, and the result was shown in FIG.2.

In FIG.2, the homogenization was performed at a low working pressure of 100 bar for 3 cycles to obtain a suspension with a particle size of 35 µm (D90). The homogenization was performed for 3 cycles at an initial working pressure of 100 bar, and then performed for 2 cycles at a medium pressure of 300 bar increased from 100 bar to obtain a suspension with a particle size of 20 µm (D90). The homogenization was performed for 3 cycles at an initial working pressure of 100 bar, and then performed for 2 cycles at a high pressure of 500 bar increased from 100 bar to obtain a suspension with a particle size of 10 µm (D90).

The experimental results showed that for the obtained active pharmaceutical ingredient particles, the particle size and the sedimentation rate were decreased, and the stability was improved, when the working pressure of the high-pressure homogenizer was increased.

### Test Example

The particle size distribution was measured by a laser granulometer, such as by light scattering or laser diffraction techniques. In order to determine the particle size, for example, the powder was loaded into a laser diffraction spectrometer through a dispersion unit. The test method is described in detail as follows:
Equipment: Mastersizer laser granulometer
Product refraction coefficient: 1.59
Absorption coefficient: 0.1
Refractive coefficient of dispersion medium: 1.33
Analysis mode: general-purpose
Measuring time: 10 s
Shading: 10% to 20%

The instrument was set up according to the above parameters. A sample was taken and reconstructed with 1 mL to 3 mL of water to prepare a suspension. The purified water was then added to dilute 10 to 50 times the original volume of the solution. An appropriate amount of the treated sample was added to the injection cell. The addition of the sample should be performed under even and continuous stirring. After the cycle was stable for 1 min, the cycle was performed 3 times for measurement and the average value was taken.

The above description is only the preferred Examples of the present application, and is not intended to limit the present application. Any modifications, equivalent substitutions, improvements, and the like made within the spirit and principles of the present application should be included within the scope of the present application.

## Claims

1. A vortioxetine pamoate powder for injection consisting of vortioxetine pamoate and pharmaceutically acceptable excipients.

2. The powder for injection according to claim 1, wherein the pharmaceutically acceptable excipients comprise a suspending agent, wherein the suspending agent is preferably selected from the group consisting of glycerol, arabic gum, agar, tragacanth gum, methyl cellulose, sodium carboxymethyl cellulose, hydroxypropyl cellulose, povidone, glucan, carbopol, and aluminum monostearate, further preferably, the suspending agent is sodium carboxymethyl cellulose.

3. The powder for injection according to claim 2, wherein the suspending agent accounts for 2.3% to 13.8% (w/w), preferably 6.9% to 10.8% (w/w) of the total mass of the powder for injection.

4. The powder for injection according to claim 1, wherein the pharmaceutically acceptable excipients comprise a freeze-drying protective agent selected from the group consisting of sucrose, trehalose, lactose, xylitol, glucose and mannitol, preferably mannitol.

5. The powder for injection according to claim 4, wherein the freeze-drying protective agent accounts for 4.6% to 35.0% (w/w), preferably 20.7% to 32.5% (w/w) of the total mass of the powder for injection.

6. The powder for injection according to claim 1, wherein the pharmaceutically acceptable excipients comprise an osmotic pressure adjuster selected from the group consisting of sodium chloride, potassium chloride, mannitol, glucose and borax, preferably mannitol.

7. The powder for injection according to claim 1, wherein the pharmaceutically acceptable excipients comprise 20.7% to 32.5% (w/w) mannitol of the total mass of the powder for injection as a freeze-drying protective agent and a osmotic pressure adjuster.

8. The powder for injection according to claim 1, wherein the mass percentage of the vortioxetine pamoate is 54.0% to 72.4%, based on total mass of the powder for injection.

9. The powder for injection according to claim 1, comprising about 235.5 mg to 471.0 mg of vortioxetine pamoate monohydrate, wherein the amount of sodium carboxymethyl cellulose is 6.9% to 10.8% (w/w), and the amount of mannitol is 20.7% to 32.5% (w/w).

10. The powder for injection according to claim 1, wherein the pharmaceutical excipients do not comprise a wetting agent.

11. The powder for injection according to claim 1, wherein the pharmaceutical excipients do not comprise Tween.

12. The powder for injection according to claim 1, wherein the vortioxetine pamoate has a particle size of 1 µm to 40 µm (D90), preferably 1 µm to 20 µm (D90), more preferably 1 µm to 10 µm (D90).

13. A preparation method of a vortioxetine pamoate powder for injection, comprising the following steps:
(1) preparation of a diluent solution: heating a water for injection, adding excipients under stirring, stirring until being completely dissolved and filtering;
(2) a primary mixing: weighing vortioxetine pamoate monohydrate powders, slowly adding into the diluent solution obtained in step (1) under stirring, and performing a high-shear dispersion to obtain a primary mixed solution;
(3) a secondary mixing: feeding the primary mixed solution obtained in step (2) into a homogenizer, performing a homogenization to obtain a suspension having a particle size of 1 µm to 40 µm (D90), filling and half-stoppering to obtain a filled sample; and
(4) freeze-drying: placing the filled sample obtained in step (3) into a dryer, freeze-drying under set freeze-drying parameters, and stoppering.

14. The preparation method according to claim 13, wherein the particle size is 1 µm to 20 µm (D90), preferably 1 µm to 10 µm (D90).

15. The preparation method according to claim 13, wherein the freeze-drying in step (4) comprises the following steps:
(1) freezing the filled suspension of the vortioxetine pamoate to -40°C;
(2) performing a primary drying at a temperature lower than about 0°C under vacuum; and
(3) performing a secondary drying at a temperature higher than about 0°C.
